# EUROPEAN PATENT APPLICATION

(11) **EP 1 147 742 A2**
(43) Date of publication of application: **24.10.2001**
(21) Application number: 01303581.1
(22) Date of filing: 19.04.2001
(51) Int. Cl.: A61B 8/06, A61B 5/026

(54) **Ultrasonic blood flow sensing apparatus**

(30) Priority: 19.04.2000 GB 0009741
(71) Applicant: Skidmore, Robert, Stoke Bishop, Bristol BS9 1TV (GB)
(72) Inventor: Skidmore, Robert, Stoke Bishop, Bristol BS9 1TV (GB)
(74) Representative: Harrison, Ivor Stanley

(57) **Abstract**

A housing for a sensor apparatus for investigating characteristics of a blood vessel comprises a base for holding one or more sensor components, at least one appendage joined to the base and capable of being attached to the blood vessel by means of thread, and an attachment point for pulling means, wherein the at least one appendage is shaped or fabricated so as to allow the pull-removal of the housing from the blood vessel without removal of the thread from the blood vessel.

Sensor apparatus for investigating the characteristics of a blood vessel comprises means for transmitting signals into the blood vessel along a first path and detecting signals returned from at least one pair of locations along the first path, wherein the apparatus further comprises means for determining from the signals whether there is a blood vessel wall between the locations of each pair.

## Description

This invention relates to apparatus and methods for sensing parameters, especially blood flow rate in a blood vessel.

It is known to transmit ultrasound into a blood vessel and measure the Doppler shift of ultrasound waves scattered by the blood flow. Taking the example of a red blood cell, waves scattered from the blood cell towards a receiver will experience a Doppler shift dependent upon the component of the velocity of the blood cell in the direction of the receiver. In other words, the velocity of scatterers in a blood flow can be deduced from the Doppler shift imparted to the scattered waves. If the size of the cross-section of the stream of flowing blood is known or can be deduced, then, given the flow velocity, the flow rate can be calculated. A knowledge of the rate of blood flow is desirable in many (e.g. medical) situations.

It is desirable to make accurate measurements of such flow rates. To increase the accuracy of a measurement made using a Doppler technique, it is desirable to measure flow velocity at different points across the blood flow to estimate the variation of flow velocity within the flow. Measurements of flow velocity at different locations across the flow can be made by pulsing the ultrasound transmitter. At a given instant in time, the signal acquired by the receiver corresponds to ultrasound waves being scattered from the present location of the transmitted pulse. Therefore, by determining a start and an end time for reception, i.e. by time-gating the received signal, the location from which the scattered ultrasound waves are received may be dictated. Several locations on the transmission path across the blood flow can be examined by "multi-gating" the received scattered ultrasound waves.

Having determined the velocity profile within the blood flow, an average flow velocity may be determined. To derive the flow rate, a knowledge of the cross sectional area of the flow is required. A conventional method of measuring this cross sectional area involves scanning a gate across the blood flow and seeking the points at which the Doppler shift of the received scattered ultrasound waves first appears and then eventually disappears.

It is an object of the invention to provide sensing apparatus and methods of improved accuracy.

According to one aspect, the invention provides a method of investigating the characteristics of a blood vessel comprising transmitting signals into the blood vessel along a path and detecting signals returned from a least one pair of locations along the path, wherein the returned signals are examined to determine whether there is a blood vessel wall between each pair of locations.

According to another, and related, aspect of the invention, there is provided apparatus for performing the above method.

The invention thus provides a method of locating a blood vessel wall, which is capable of improved accuracy. In turn, this leads to a more accurate method of measuring the cross-section of a blood vessel.

Preferably, there are two paths along which the signals are transmitted, the paths being separated by a predetermined angle. In this way, the angle of the paths relative to the vessel and the vessel diameter may be calculated.

In a preferred embodiment, signals from a plurality of pairs of locations along the transmission path are detected so as to notionally sweep a pair of locations (or time-gates) along the path. The locations in each pair may abut one another. The selected pairs of locations may be chosen so that the notional sweeping action is a continuous sweeping action.

Advantageously, signals returning from a pair of locations are compared on the basis of at least one of their energy and frequency. Preferably, the transmitted signal is an ultrasonic signal.

In a particularly preferred embodiment, the method comprises locating both vessel walls and determining their separation. In this way, it is possible to determine the boundaries of, for example, the blood flow in the vessel. With knowledge of the boundaries of such a flow, the flow rate may be determined.

According to another aspect, the invention provides a method of aligning a sensor relative to a blood vessel, comprising providing a sensor which transmits a signal along a path in a plane, positioning the sensor so that the path intersects the blood vessel, adjusting the position of the sensor relative to the blood vessel as signals returned from the blood vessel are detected and determining from the detected signals a desired orientation of the sensor relative to the vessel in which the axis of the vessel lies in the plane.

According to a further, and related, aspect, the invention also provides apparatus for performing the above method.

Thus the invention facilitates the improvement of the alignment of a sensor with a blood vessel to improve the accuracy of measurements made on the latter.

Advantageously, the signals returning from the vessel are detected for several different orientations of the sensor relative to the vessel. These detected signals may then be used to determine the location of the centre of the vessel relative to the sensor path and permit the sensor to be realigned so that the path substantially intersects the centre of the vessel.

In a preferred embodiment, signals returned from at least one location on the path are detected and the sensor is realigned until at least one property of the returned signals is optimised. In one case, signals returned from just one location on the path are detected and the sensor is realigned until, for at least one property, the value thereof for signals returned from said one location is optimised. In an alternative arrangement, signals returned from a plurality of locations on the path are detected and the sensor is realigned until, for at least one property, the total of the values thereof for signals returned from the locations is optimised.

According to a yet further aspect, the invention provides sensor apparatus for measuring characteristics of a blood vessel, comprising sensing means for detecting at least one property of a blood vessel, transmission means for conveying signals between sensing means and a processing means for processing, and interference suppressing means for reducing the appearance of distortion or interference in the signals conveyed by the transmission means.

The invention thus provides for more accurate sensing, in that the signals (including the raw measurement data) exchanged between the sensing means and the processing means are less likely to be distorted.

In a preferred embodiment, the transmission means provides two signal paths (such as a pair of twisted wires) for conveying signals between the sensing means and the processing means, the two paths being arranged such that they experience substantially the same interference and/or distortion. This provides that the value of the difference between the signals on the two paths is less effected by interference and/or distortion.

Advantageously, the sensor apparatus further comprises isolating means for preventing potentially damaging signals being conveyed between the processing means and the sensing means or the living body via the transmission means. This provides for the protection of the equipment and the test subject. Where the transmission means comprises said aforementioned two paths, the isolating means ideally comprises means for providing a signal indicative of the difference between the signals on the paths of the transmission means. For example, the isolating means may be a transformer, possibly of the single-turn, air-gap kind.

According to a different aspect, the invention provides sensor apparatus for measuring characteristics of a blood vessel, comprising a sensor for transmitting signals into and for receiving signals returned from the blood vessel and means for fixing the apparatus to the exterior of the blood vessel.

The invention thus provides sensor apparatus suited to application to a blood vessel, such as the ascending aorta.

The sensor apparatus may include a second sensor at a fixed angle relative to the first. The sensor apparatus may comprise a housing or body having a surface which is shaped to confirm, or able to conform, to the exterior surface of a blood vessel. The sensor apparatus may have eyelets to enable it to be stitched to a blood vessel.

In a preferred embodiment, the sensing means comprises a piezoelectric transducer.

A further aspect of the invention provides a housing for a sensor apparatus for measuring characteristics of a biological structure, the housing comprising a base for holding one or more sensor components, at least one appendage joined to the base and capable of being attached to the structure by means of thread, and an attachment point for pulling means, wherein the at least one appendage is shaped or fabricated so as to allow the pull-removal of the housing from the structure without removal of the thread from the structure.

The housing preferably also includes a separate cover to further protect the other elements of the housing and the sensor components from biological material. The housing is preferably constructed from a plastics material.

Preferably, the housing has an even number of appendages arranged substantially symmetrically about the pull-removal axis. In certain embodiments, four appendages are so arranged.

In one embodiment, the or each appendage comprises a shaft portion extending substantially parallel to the pull-removal axis and a knob portion, of greater thickness than the shaft portion, at the end of the shaft portion distal to the base-appendage joint and opposite the pull-removal direction. In use, thread is sewn around the shaft portion so as to attach it and the base to the biological structure with thread loops. The knob portion prevents the shaft portion from sliding out of the thread loops during normal use of the housing. However, when a moderate pulling force is applied, via the pulling means attachment point, the knob portion is able to deform the thread and/or the sewn biological structure so as to be able to slide through the thread loops.

In an alternative embodiment, the or each appendage comprises a flexible shaft which, when a moderate pulling force is applied, via the pulling means attachment point, bends so as to lie in a direction substantially parallel to the pull-removal axis and hence slide through the thread loops .

In a further alternative embodiment, the or each appendage is capable of retraction into or against the base such that, when a moderate pulling force is applied, via a pulling means attachment point, it or they retract into or against the base allowing it or them to slide through the thread loops.

In a preferred embodiment, the sensor components comprise at least one signal transmitter and/or at least one signal receiver. Preferably, the transmitted signal is an ultrasonic signal. There may be two combined signal transmitters and receivers arranged at a fixed angle relative to each other such that the sensor apparatus can be more conveniently used to measure flow of, for example, blood through the biological structure by a Doppler technique. In such an embodiment, the housing may be advantageously shaped so as to conform to the exterior surface of a blood vessel, such as the aorta.

Preferably, the pulling means comprise a sleeve of plastics material which also serves as a covering insulator for electrical communication means between the transmitter and/or receiver and external devices connected to the sensor apparatus. When the sensor apparatus is used *in vivo*, the pulling means may pass along the inside of a conduit which extends from the region of the biological structure to the extracorporeal environment. Thus, the housing may be pulled form the biological structure by pulling the extracorporeal end of the pulling means. The extracorporeal end of the conduit will usually be fitted with a stopper through which the pulling means is passed. Advantageously, the pulling means has a docking mark at a point along its length such that a user pulling the extracorporeal end is able to determine when the housing has been safely pulled into the conduit, following which the conduit can be removed from the body.

When the sensor apparatus is used to interrogate the vessels proximal to the heart, such as the aorta, the conduit may be a chest draining tube as would be conventionally fitted following thoracic surgical procedures. The use of the housing of the present invention avoids the need for further surgical intervention to remove the sensor apparatus following monitoring of a patient post surgery. Conventionally, biodegradable suture thread is used and hence the fact that the thread loops will be left on the biological structure should pose minimal threat to the patient.

By wayof example only, certain embodiments of the invention will now be described with reference to the accompanying drawings, in which:
Figures 1 and 2 illustrate the application of time-gated Doppler ultrasound measurements to a blood vessel;
Figure 3 is a plot illustrating the processing performed by the apparatus used in Figures 1 and 2;
Figures 4 and 5 schematically illustrate a method of aligning a Doppler ultrasound sensor a blood vessel;
Figure 6 illustrates schematically an ultrasound sensor applied to a test body;
Figures 7 to 9 illustrate schematically an ultrasonic sensor;
Figure 10 shows a perspective view from above of an embodiment of the sensor apparatus housing of the present invention;
Figure 11 shows the housing of Figure 10 provided with a cover; and
Figure 12 shows a schematic sectional view along the central longitudinal axis of the housing of Figure 11.

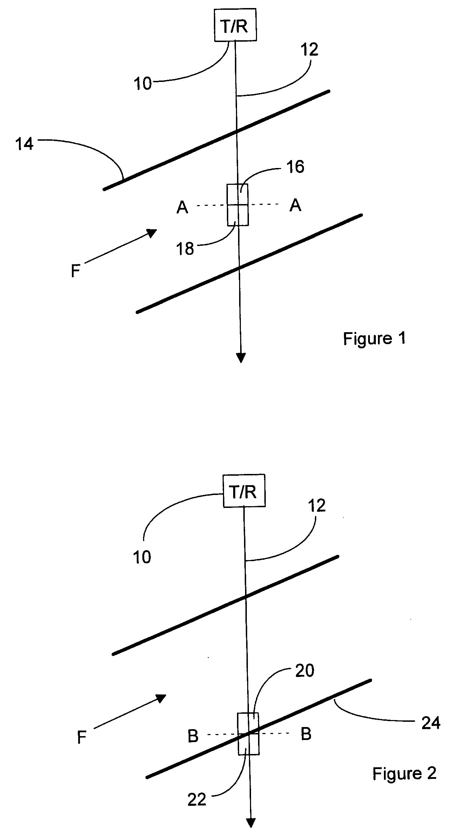

In Figure 1, a sensor 10 comprising an ultrasonic transmitter and an ultrasonic receiver transmits ultrasonic pulses along path 12 which intersects an artery 14. There is a blood flow in the direction of the arrow F in the artery 14. The transmitted ultrasonic pulses are scattered by the blood flow. Sensor 10 acquires ultrasonic signals scattered back along path 12 to sensor 10. As is well known in the art, a single piezoelectric transducer can be used for both transmitting and receiving ultrasonic signals.

The reception of the scattered ultrasonic signals by sensor 10 is time-gated to examine particular locations along path 12. The acquisition of returned signals at sensor 10 is timed so that the returned signals correspond to ultrasonic waves scattered upon the transmitted pulse reaching the desired locations on the transmission path 12. Two time-gated locations 16 and 18 are shown in figure 1. The locations abut one another on the transmission path 12, as indicated by line A-A.

The energy of an acquired signal returning from a given location on path 12 is dependent upon the number of scattering particles in the blood flow at that particular location. In addition, the frequency of the signals returning from a given location will experience a Doppler shift which is dependent upon the vector velocity of the flow at that particular location relative to the reception path (in this case path 12). Thus, for a given location along path 12, there are two specific measurable quantities of the returning signals: energy and Doppler shift. There is also a third, derivable quantity: the product of the energy and the Doppler shift. These three quantities can be regarding as indicator parameters. If an indicator parameter is deduced for each of locations 16 and 18, then the ratio, R, of the indicator parameter values for locations 16 and 18, as shown in figure 1, will be near unity. The ratio R will tend to unity as the extent of locations 16 and 18 along path 12 about line A-A (governed by the time-gating of the sensor 10) tends to zero.

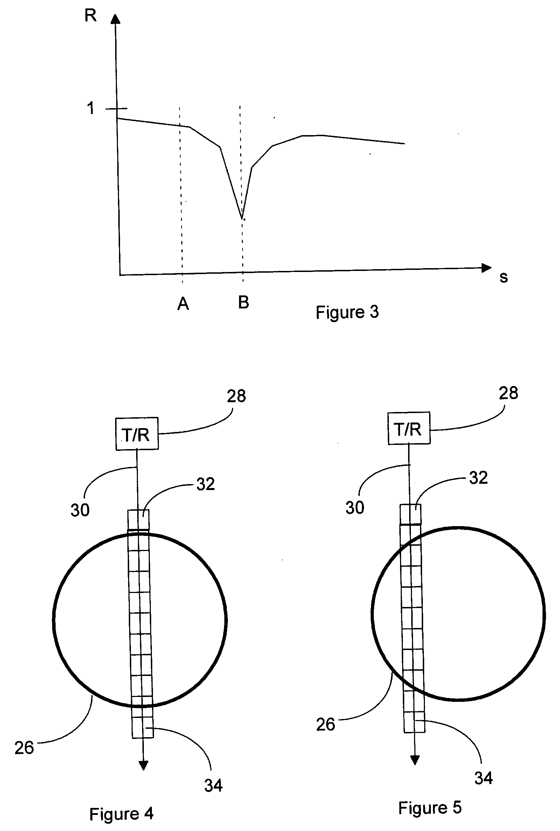

A plot of the ratio R for distance S from the sensor 10 is shown in figure 3. The value of R for the distance along path 12 corresponding to line A-A in figure 1 is indicated A in figure 3.

Figure 2 illustrates the situation where sensor 10 is time-gated to examine two adjacent locations 20 and 22 which meet at a point on path 12 indicated by line B-B. It should be noted also that the intersection of line B-B and path 12 lies on the artery wall 24 furthest from sensor 10. It will be appreciated that location 20 lies substantially within the blood flow F and that location 22 lies substantially outside the artery. Thus, the energy scattered from location 20 towards sensor 10 will be considerably different to the amount of energy scattered back from location 22. Similarly, since location 22 is outside the blood flow F, the Doppler shift of signals scattered to sensor 10 from this location will be approximately zero. On the other hand, the signals scattered to sensor 10 from location 20 will exhibit a significant Doppler shift. Considering, therefore, the ratio for any of the aforementioned indicator parameters (energy, Doppler shift or product thereof), it will deviate significantly from unity at point B-B on path 12. Point B-B is illustrated by a notch at B in the plot of R in figure 3.

In operation, the sensor 10 is arranged to time-gate the acquisition of returning signals on path 12 so as to scan a pair of adjacent reception locations along path 12. Effectively, this corresponds to scanning notional line A-A of figure 1 (or notional line B-B of figure 2) along path 12. The sensor is arranged to monitor the ratio R of the values of an indicator parameter from each of the adjacent reception locations. When the ratio R deviates significantly or rapidly from unity, it is determined that the position of a wall of the artery (such as 24) has been encountered. Hence, the separation of the arterial walls may be determined, allowing subsequent calculation of the blood flow rate.

It is particularly advantageous to base the ratio R under investigation upon an indicating parameter which has a Doppler shift dependence. Because the Doppler shift falls to zero beyond the blood flow, this means that the interfaces located by monitoring the ratio R are then not the walls of the artery, but the true boundaries of the blood flow therein, hence allowing a yet more accurate measure of flow rate (there may be a region adjacent the arterial walls where there is an insignificant net blood flow).

In order to calculate the blood flow rate, it is necessary to calculate the velocity of the flowing blood from the measured Doppler shift. The formula for this calculation is well known and has a dependence upon the angle between the reception path of the receiver (e.g. 12 in Figure 1) and the vectorial velocity of the blood flow. It is desirable to know this angle (the "beam vessel angle") accurately.

When a transmitter/receiver is applied to a blood vessel, the beam vessel angle may not be known to a high degree of accuracy. This problem can be overcome by providing two transmitter/receivers in the sensor, whose transmission/reception paths are separated by a fixed angle β.

Using such a sensor (an example of which will be described later), each transmitter/receiver can be used independently to measure the distance between the blood vessel walls by the method described with reference to Figures 1 and 2. For each transmitter/receiver, the measured distance represents an effective vessel diameter. Assuming that the blood vessel is cylindrical, the beam vessel angle can be calculated for each transmitter/receiver from the angle β and the two effective diameters.

An average velocity for the blood vessel can be calculated from the discrete velocities calculated for each of a series of time gates across the blood vessel. The discrete velocities must be given a weighting in the averaging process which is dependent upon their radial position within the blood vessel's cross-section. This weighting process is simplified if the transmission path of the (or each) transmitter/receiver being used to measure the discrete velocities intersects the centre of the blood vessel. The process by which this intersection can be achieved will now be described.

Figure 4 illustrates, in cross-section, a blood vessel 26 which is assumed to have a circular cross-section. A sensor 28, is used to investigate the blood flow rate within the blood vessel 26. The sensor 28 comprises a piezoelectric transmitter/receiver which transmits ultrasonic pulses along path 30, and receives returned signals travelling in the opposite direction on the same path. The sensor 28 is time-gated to acquire returning signals which correspond to a sequence of consecutive locations along path 30. Locations 32 and 34 are the terminal locations of this sequence. In figure 4, the transmission path 30 intersects the central axis of blood vessel 26. Thus, a maximum number of the acquisition locations 32 to 34 will be within blood vessel 26. Thus, for any of the aforementioned indicator parameters, the cumulative total of the values for all of the locations 32 to 34 will assume a maximum value for the orientation shown in figure 4.

In Figure 5, the transmission path 30 no longer intersects the central axis of blood vessel 26. Thus, a fewer number of acquisition locations 32 to 34 fall within blood vessel 26 than in the orientation shown in figure 4. Thus, the cumulative total of the values of a given indicator parameter for acquisition locations 32 to 34 will be lower in the Figure 5 orientation than in the Figure 4 orientation. The perpendicular distance between the central axis of blood vessel 26 and transmission path 30 can be said to quantify a "misalignment" of the sensor 28 and the blood vessel 26. By moving sensor 28 in the plane of the diagram to maximise the cumulative total of the values of an indicator parameter totalled across the acquisition locations 32 to 34, misalignment can be minimised.

The minimisation of the cumulative total of an indicator parameter may be achieved manually by allowing a user to monitor the cumulative total of the indicator parameter in question whilst repositioning the sensor 28. Alternatively, the sensor 28 may contain processing circuitry which monitors the cumulative total of the indicator parameter in question as the sensor 28 is repositioned, the processing circuitry noting a maximum value which indicates a minimum misalignment.

In the method described with reference to Figures 4 and 5, a consecutive sequence of gates is used. In an alternative arrangement, the sensor 28 could be arranged to acquire returning signals from a single location along path 30 within the blood vessel 26. The previously-defined misalignment could be minimised by examining, as the sensor 28 is repositioned, the variation of a Doppler-shift-based indicator parameter of signals returned from the single location on the path 30. A Doppler-shift-based indicator parameter will have a dependence upon the velocity of the flow at the location sensed. The distribution of velocities within the blood vessel 26 allows these determinations to be made. The blood flow velocity is highest on the central axis of the blood vessel 26 and decreases radially to zero at the blood vessel walls (assuming, amongst other things, that the artery has a circular cross-section).

When the transmission path is aligned with the centre of the blood vessel, the sensor is time-gated to acquire discrete velocities along the nearest half of the vessel's diameter. These velocities are then weighted in an averaging process by π (rₒ²-rᵢ²) where rₒ is the maximum, or outer, radius specified by the corresponding time-gated location and rᵢ is the minimum, or inner, radius for the corresponding time gate.

Once the average flow velocity in the vessel has been calculated, the flow rate in the vessel can be determined using a knowledge of the vessel diameter. The vessel diameter can be calculated (in the two transmitter/receiver system mentioned above) using the two effective diameters and the separation angle β.

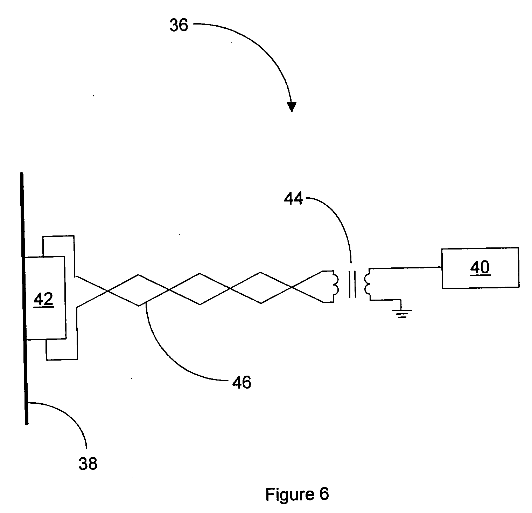

Figure 6 illustrates schematically an ultrasonic sensor 36 applied to the surface of a test body 38. A processing unit 40 provides driving signals to piezoelectric crystal 42 which, in turn, transmits ultrasonic signals into the test body 38. The piezoelectric crystal 42 also receives returned ultrasonic signals from within body 38, and transduces these to electric signals which are transmitted to processing unit 40. Signals are exchanged between piezoelectric crystal 42 and processing unit 40 via a transformer 44. The primary purpose of transformer 44 is to isolate, on the one hand, the processing unit 40 from, on the other hand, the piezoelectric crystal 42 and the test body 38. The transformer 44 therefore blocks the transmission of signals carrying of sufficient energy to damage the apparatus or the test body 38.

The piezoelectric crystal 42 is connected to the transformer by a twisted pair of wires 46. Due to their spatial intimacy, any interference affecting a point on one of the wires 46 also affects the adjacent point of the other one of the wires 46. When piezoelectric crystal 42 transduces a returned ultrasonic signal into an electrical signal, this electrical signal is represented by a voltage difference between the two wires of the twisted pair 46 interference affecting the twisted pair will not affect the difference between the signals conveyed on the pair of wires. The transformer 44 transfers to processing unit 40 a signal proportional to the difference between the signals on the twisted pair 46. Therefore, any interference occurring between piezoelectric crystal 42 and the transformer 44 is removed from signals passing to the processing unit 40. The combination of the twisted pair 46 and the transformer 44 provides useful interference suppression in addition to electrical isolation.

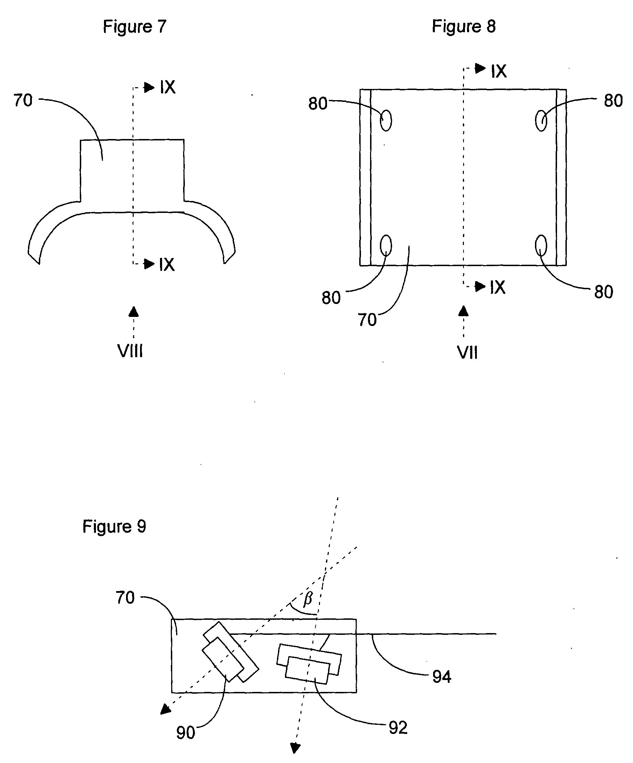

A two transmitter/receiver sensor 70 (as mentioned above) is shown in Figures 7, 8 and 9. The sensor 70 is designed to be applied to the exterior of a blood vessel, for example the ascending aorta adjacent the heart.

The sensor 70 has a concave face which conforms to the exterior of the subject blood vessel. The edges of the concave face provide pliable wings allowing a close fit to the subject blood vessel. The sensor 70 has holes 80 through the wings permitting it to be stitched onto the subject blood vessel after it has been optimally aligned relative thereto (e.g. using the process described with reference to Figures 4 and 5).

The sensor 70 contains two piezoelectric transducers 90 and 92, each of which constitutes an ultrasonic transmitter/receiver. The transducers 90, 92 project ultrasound through the concave face of the sensor 70 into the subject vessel. The transducers 90 and 92 are held within sensor 70 such that their transmission paths adopt a fixed angle β relative to one another, thus allowing accurate determination of the beam vessel angle and true vessel diameter by using the earlier-described two transmitter/receiver method.

A twisted pair of wires extends from each transducer 90, 92 and these are combined into a single cable 94 for connection to processing equipment via an isolating transformer. The sensor 70 is a single-use, disposable unit.

Although the invention has been described herein with reference to investigating blood vessels, it will be apparent to the skilled person that the invention is equally applicable to the investigation of other fluid bearing conduits.

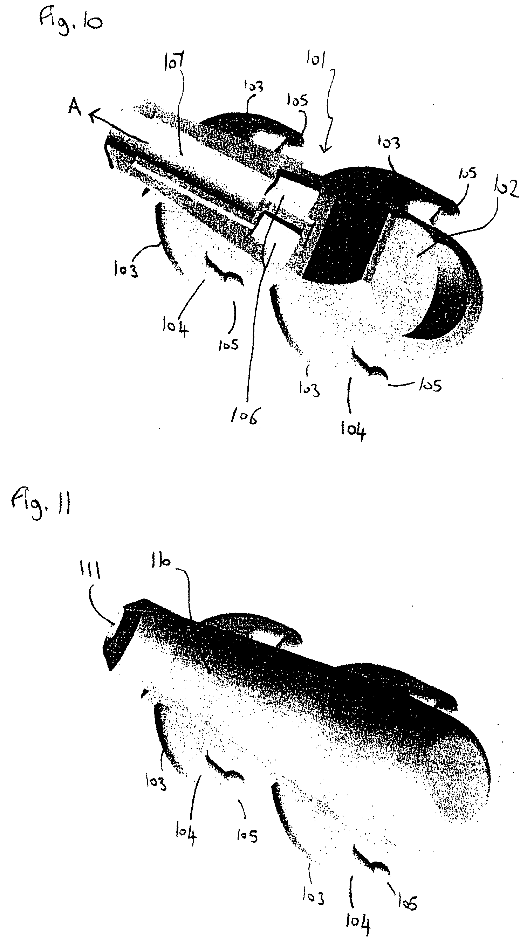

Turning now to Figure 10, there is shown a housing for a sensor apparatus and according to the present invention. The housing comprises a base, generally indicated 101, which has a sub-housing 102 for holding sensor components . Projecting from the base are four arms 103. When the underside (as shown in the present view) is placed against a biological structure, such as a blood vessel, suture thread can be sewn around the shaft portions 104 of the arms 103 and through the wall of the biological structure, thus fixing the base 101 to the structure. During normal use of the sensor apparatus, the base is prevented from release from the thread loops by the bulbous ends 105 of the arms 103. An attachment point for pulling means (not shown) is fabricated as part of the base 101. The attachment point comprises a pair of shoulders 106 which, when pulling means bearing an end wider than the gap between the shoulders 106 is inserted, translate pulling force applied to the pulling means in the direction A to the arms 103 so as to cause the bulbous ends 105 to pass through the thread loops when the pulling force is sufficient.

Electrical connections to the device are terminated in the region of the sub-housing 102 so as to allow the sensor components to communicate with external devices. Conductive wires enter the housing along the channel 107 and pass between the shoulders 106. Advantageously, insulating material surrounding the wires, which will generally comprise a sleeve of plastics material, will also comprise the pulling means. Further attachment points for the pulling means can clearly be provided in addition to the shoulders 106. For example, the insulating material and wires can be frictionally held within the channel. In addition, the electrical terminations in the sub-housing 102 will also provide an attachment point for translation of the pulling force although it is not desirable to have this as the only attachment point.

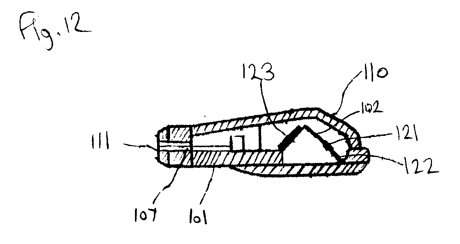

In Figure 11, the housing further comprises a cover 110 intended to provide additional isolation of the base and sensor components from the biological environment into which they will be placed in use. The cover essentially seals the housing apart from the orifice 111 formed by the channel 107 and the corresponding surface in the cover.

Figure 12 shows an example of an arrangement of sensor components within the sub-housing 102 of the base 101. In the embodiment shown, the sensor apparatus is intended to be used for Doppler ultrasound flow measurements in a blood vessel. Thus, the components comprise two piezoelectric transducers 121 and 122 positioned at different angles. These transducers are capable of both transmitting and receiving ultrasound energy. A circuit board 123 is provided to enable control of the transducers. The cover 110 can be seen to provide additional protection to the sensor components over and above that offered by the sub-housing 102.

## Claims

1. A housing for a sensor apparatus for investigating characteristics of a blood vessel, the housing comprising a base for holding one or more sensor components, at least one appendage joined to the base and capable of being attached to the blood vessel by means of thread, and an attachment point for pulling means, wherein the at least one appendage is shaped or fabricated so as to allow the pull-removal of the housing from the blood vessel without removal of the thread from the blood vessel.

2. A housing according to claim 1 wherein an even number of appendages are arranged substantially symmetrically about the pull-removal axis.

3. A housing according to claim 1 or claim 2 wherein the or each appendage comprises a shaft portion extending substantially parallel to the pull-removal axis and a knob portion, of greater thickness than the shaft portion, at the end of the shaft portion distal to the base-appendage joint and opposite the pull-removal direction.

4. A housing according to any preceding claim wherein the or each appendage comprises a flexible shaft which, when a moderate pulling force is applied via the pulling means attachment point, bends so as to lie in a direction substantially parallel to the pull-removal axis and hence slide through the thread loops.

5. A housing according to any preceding claim wherein the or each appendage is capable of retraction into or against the base such that, when a moderate pulling force is applied via the pulling means attachment point, it or they retract into or against the base, allowing it or them to slide through the thread loops.

6. A housing according to any preceding claim wherein the sensor components comprise at least one signal transmitter and/or at least one signal receiver.

7. A housing according to claim 6 wherein the transmitted signal is an ultrasonic signal.

8. A housing according to claim 6 or claim 7 wherein the sensor components comprise two combined signal transmitters and receivers arranged at a fixed angle relative to each other.

9. A housing according to any preceding claim further comprising pulling means which comprise a sleeve of plastics material which also serves as a covering insulator for electrical communication means between the transmitter and/or receiver and external devices connected to the sensor apparatus.

10. A housing according to claim 9 further comprising a conduit, through which the pulling means are passed, which extends in use from the region of an *in vivo* blood vessel to the extracorporeal environment.

11. A housing according to claim 10 wherein the pulling means has a docking mark at a point along its length such that a user pulling the extracorporeal end is able to determine when the housing has been safely pulled into the conduit.

12. A housing according to claim 10 or claim 11 wherein the conduit is a chest draining tube for use following thoracic surgery.

13. Sensor apparatus for investigating the characteristics of a blood vessel, comprising means for transmitting signals into the blood vessel along a first path and detecting signals returned from at least one pair of locations along the first path, wherein the apparatus further comprises means for determining from the signals whether there is a blood vessel wall between the locations of each pair.

14. Apparatus according to claim 13, further comprising means for examining the returned signals to determine the positions of both blood vessel walls along the first path to calculate a first effective wall separation.

15. Apparatus according to claim 13 or 14, further comprising means for transmitting signals into the blood vessel along a second path at a predetermined angle relative to the first path, means for detecting signals returned from at least one pair of locations on the second path and means for determining from the returned signals whether there is a blood vessel wall between the of locations of each pair.

16. Apparatus according to claim 14, further comprising means for examining the returned signals to determine the positions of both blood vessel walls along the second path to calculate a second effective wall separation.

17. Apparatus according to claim 16, further comprising calculating means for using the first and second effective wall separations to calculate at least one of the angle of the first and second paths relative to the vessel, and the cross-sectional area of the vessel.

18. Apparatus according to any one of claims 13 to 17, wherein the detecting means is arranged to detect signals returning from a plurality of pairs of locations along the respective transmission path so as notionally to sweep a pair of locations along said path.

19. Apparatus according to any one of claims 13 to 18, wherein the means for determining whether there is a blood vessel wall between the pair of locations comprises means for comparing at least one property of the signals returned from the two paired locations.

20. Sensor apparatus including means for aligning the apparatus relative to a blood vessel, the apparatus comprising means for transmitting signals along a path in a plane, means for adjusting the position of the transmitting means, means for detecting signals returned from the blood vessel as the position of the transmitting means relative to the blood vessel is adjusted and means for determining from the detected signals a desired orientation of the transmitting means relative to the vessel in which the axis of the vessel lies in the plane.

21. Apparatus according to claim 20, wherein the detecting means is arranged to detect signals returned from at least one location along the path.

22. Apparatus according to claim 20, wherein the detecting means is arranged to detect signals returned from just one location on the path and the determining means is arranged to distinguish the desired orientation as that orientation in which at least one predetermined parameter of the signals returning from said location is optimised.

23. Apparatus according to claim 20 or claim 21, wherein the detecting means is arranged to detect signals returning from a plurality of locations along the path and the determining means is arranged to distinguish the desired orientation as that orientation in which the detected signals indicate that the number of locations falling within the blood vessel is optimised.

24. Apparatus according to claim 20 or claim 21, wherein the detecting means is arranged to detect signals returned from a plurality of locations along the path and the determining means is arranged to distinguish the desired orientation as that orientation signified by the optimisation of the cumulative total of a property of the signals returned from the locations.

25. Apparatus according to any one of claims 13 to 24, wherein the returned signals are examined on the basis of at least one of their energy or frequency.

26. Apparatus according to any one of claims 13 to 25, wherein the transmitted signals are pulsed.

27. Apparatus according to claim 25 or claim 26, wherein the means for detecting signals returned from a location on their transmission path comprises means for timing the acquisition of the returned signals to correspond to the transmitted pulses reaching said location.

28. Apparatus according to any one of claims 13 to 27, wherein the transmitted signals are ultrasound signals.

29. Apparatus according to any one of claims 13 to 28, further comprising means for measuring the velocity of blood within the vessel on the basis of the Doppler shift experienced by signals returned from the vessel.

30. Apparatus according to claim 29, comprising means for computing an average velocity from velocities measured across the vessel.

31. Apparatus according to claim 30, comprising means for weighting the velocities in the averaging process in accordance with their position across the vessel.

32. Apparatus according to any of claims 16 to 31, comprising means for using a velocity for the blood in the vessel to determine a flow rate for blood in the vessel.

33. Sensor apparatus according to any of claims 13 to 32, further comprising interference suppressing means for reducing the appearance of distortion or interference in the signals conveyed by the transmission means.

34. Sensor apparatus according to claim 33, wherein the transmission means provides two paths for conveying signals between the sensing means and the processing means, the two paths being arranged such that they experience substantially the same interference and/or distortion.

35. Sensor apparatus according to claim 34, wherein the transmission means comprises a pair of twisted wires.

36. Sensor apparatus according to any one of claims 33 to 35, further comprising isolating means for preventing potentially damaging signals being conveyed between the processing means and the sensing means or a living body of which the vessel is a part via the transmission means.

37. Sensor apparatus according to claim 36, when dependent on claim 34 or 35, wherein the isolating means is arranged to provide a signal indicative of the difference of the signals on the paths of the transmission means.

38. Sensor apparatus according to claim 36 or 37 wherein the isolating means is a signal transforming means.

39. Sensor apparatus according to claim 38, wherein the isolating means is a single turn, air gap transformer interposed between the sensing means and the processing means.

40. Sensor apparatus according to any one of claims 33 to 39, wherein the sensing means comprises a piezoelectric sensor.

41. Sensor apparatus according to any of claims 13 to 40, including means for fixing the apparatus to the exterior of a blood vessel.

42. Sensor apparatus according to claim 41, wherein the fixing means comprises eyelets to permit the apparatus to be stitched onto the blood vessel.

43. Sensor apparatus according to claim 41 or 42, comprising a body having a surface which conforms to the exterior of the blood vessel.

44. Sensor apparatus according to claim 43, wherein the body is pliable in the region of the surface.

45. Sensor apparatus according to any one of claims 41 to 44, comprising a second sensor for receiving signals returned from the blood vessel, the second sensor being arranged at a predetermined angle relative to the first sensor.

46. A method of investigating the characteristics of a blood vessel comprising transmitting signals into the blood vessel along a first path and detecting signals returned from at least one pair of locations along the first path, wherein the returned signals are examined to determine whether there is a blood vessel wall between each pair of locations.

47. A method according to claim 46, further comprising examining the returned signals to determine the positions of both blood vessel walls along the first path to calculate a first effective wall separation.

48. A method according to claim 46 or 47, further comprising transmitting signals into the blood vessel along a second path at a predetermined angle relative to the first path, detecting signals returned from at least one pair of locations on the second path and determining from the returned signals whether there is a blood vessel wall between the locations of each pair.

49. A method according to claim 48, further comprising examining the returned signals to determine the positions of both blood vessel walls along the second path to calculate a second effective wall separation.

50. A method according to claim 49, further comprising using the first and second effective wall separations to calculate at least one of the angle of the first and second paths relative to the vessel, and the cross-sectional area of the vessel.

51. A method according to any of claims 46 to 50, wherein signals returning from a plurality of pairs of locations are detected so as notionally to sweep a pair of locations along the respective transmission path.

52. A method according to any of claims 46 to 51, wherein determining whether there is a blood vessel wall between the locations of a pair comprises comparing at least one property of the signals returned from the two paired locations.

53. A method of aligning a sensor relative to a blood vessel, comprising providing a sensor which transmits a signal along a path in a plane, positioning the sensor so that the path intersects the blood vessel, adjusting the position of the sensor relative to the blood vessel as signals returned from the blood vessel are detected and determining from the detected signals a desired orientation of the sensor relative to the vessel in which the axis of the vessel lies in the plane.

54. A method according to claim 53, wherein signals returned from at least one location along the path are detected.

55. A method according to claim 54, wherein signals returned from just one location on the path are detected and the desired orientation is indicated by the optimisation of at least one parameter of the signals returning from said location.

56. A method according to claim 54, wherein signals returned from a plurality of locations along the path are detected and the desired orientation is signified by the detected signals indicating that the number of said locations falling within the blood vessel is optimised.

57. A method according to claim 54, wherein signals returned from a plurality of locations along the path are detected and the desired orientation is signified by the cumulative total of a property of the signals returned from the locations being optimised.

58. A method according to any of claims 53 to 57, wherein returned signals are examined on the basis of at least one of their energy and their frequency.

59. A method according to any of claims 53 to 58, wherein the transmitted signals are pulsed.

60. A method according to claim 59, wherein detecting signals returned from a location on their transmission path comprises timing the acquisition of the returned signal to correspond to the transmitted pulses reaching said location.

61. A method according to any of claims 46 to 60, wherein the transmitted signals are ultrasound signals.

62. A method according to any of claims 46 to 61, comprising measuring the velocity of blood within the vessel on the basis of the Doppler shift experienced by signals returned from the vessel.

63. A method according to claim 62, comprising computing an average velocity from velocities measured across the vessel.

64. A method according to claim 63, comprising weighting the velocities in the averaging process in accordance with their positions across the vessel.

65. A method according to any one of claims 62 to 64, comprising using a velocity for the blood in the vessel to determine a flow rate for blood in the vessel.

66. A method of investigating characteristics of a blood vessel substantially as hereinbefore described with reference to the accompanying figures.

67. Apparatus for investigating characteristics of a blood vessel substantially as hereinbefore described with reference to the accompanying figures.

68. A housing for a sensor apparatus substantially as hereinbefore described with reference to the accompanying figures.
